(19) Europäisches Patentamt
European Patent Office
Office européen des brevets

(11) **EP 3 900 610 A1**

(12) **EUROPEAN PATENT APPLICATION**
published in accordance with Art. 153(4) EPC

(43) Date of publication:
**27.10.2021  Bulletin 2021/43**

(51) Int Cl.:
**A61B 5/00** (2006.01)

(21) Application number: **19912359.7**

(86) International application number:
**PCT/CN2019/074670**

(22) Date of filing: **03.02.2019**

(87) International publication number:
**WO 2020/155169 (06.08.2020 Gazette 2020/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Huawei Technologies Co., Ltd.
Longgang
Shenzhen, Guangdong 518129 (CN)**

(72) Inventors:
• **YANG, Bin**
  **Shenzhen, Guangdong 518129 (CN)**
• **LI, Hongbao**
  **Shenzhen, Guangdong 518129 (CN)**
• **ZHANG, Jie**
  **Shenzhen, Guangdong 518129 (CN)**

(74) Representative: **Goddar, Heinz J.
Boehmert & Boehmert
Anwaltspartnerschaft mbB
Pettenkoferstrasse 22
80336 München (DE)**

(54) **ATRIAL FIBRILLATION SCREENING METHOD AND DEVICE**

(57)    A method and an apparatus (100) for detecting atrial fibrillation are provided. A BCG signal is introduced during atrial fibrillation screening, to help improve accuracy of atrial fibrillation prediction. The method for detecting atrial fibrillation includes: obtaining a pulse wave signal and an acceleration signal of a user; determining a ballistocardiogram signal and an acceleration body movement signal based on the acceleration signal, where the ballistocardiogram signal is used to represent an acceleration change of a body caused by atrial pumping, and the acceleration body movement signal is used to represent a movement acceleration change of the user; and determining, based on the pulse wave signal and the ballistocardiogram signal, whether the user has atrial fibrillation.

EP 3 900 610 A1

FIG. 2

# Description

## TECHNICAL FIELD

This application relates to the field of electronic devices, and more specifically, to a method and an apparatus for atrial fibrillation screening in the field of electronic devices.

## BACKGROUND

Atrial fibrillation (atrial fibrillation, AF), also known as AFib, is a common type of arrhythmia. Prolonged atrial fibrillation may cause serious complications, such as heart failure, hypertension, and a most serious stroke. Therefore, detection of atrial fibrillation is particularly important. During atrial fibrillation screening, if RR intervals are completely irregular within a period of time and a P wave in an electrocardiogram is deformed or disappears, it is determined as atrial fibrillation; or otherwise, it is not determined as atrial fibrillation.

Currently, atrial fibrillation of a user can be detected by using a mobile terminal. In an existing method for atrial fibrillation screening, a mobile terminal is used to detect a pulse of a user, and whether the user has a risk of atrial fibrillation is determined based on a change status of the pulse. However, the pulse can only reflect a change in a heart rate, but not in an atrial function. Therefore, accuracy of this solution for predicting atrial fibrillation is low.

## SUMMARY

This application provides a method and an apparatus for detecting atrial fibrillation. ABCG signal is introduced during atrial fibrillation screening, to help improve accuracy of atrial fibrillation prediction.

According to a first aspect, a method for detecting atrial fibrillation is provided, including:

obtaining a pulse wave signal and an acceleration signal of a user;
determining a ballistocardiogram signal and an acceleration body movement signal based on the acceleration signal, where the ballistocardiogram signal is used to represent an acceleration change of a body caused by atrial pumping, and the acceleration body movement signal is used to represent a movement acceleration change of the user; and
determining, based on the pulse wave signal and the ballistocardiogram signal, whether the user has atrial fibrillation.

Therefore, in an embodiment of this application, a PPG signal and an ACC signal are obtained, a BCG signal and an ACC body movement signal are obtained based on the ACC signal, and then whether the user has atrial fibrillation is screened based on the BCG signal and the PPG signal. Because the BCG signal can represent the acceleration change of the body caused by atrial pumping, the BCG signal is introduced during atrial fibrillation screening in this embodiment of this application, to help improve accuracy of atrial fibrillation prediction.

With reference to the first aspect, in some implementations of the first aspect, the determining, based on the pulse wave signal and the ballistocardiogram signal, whether the user has atrial fibrillation includes:

extracting a feature of the pulse wave signal, where the feature of the pulse wave signal includes at least one of Shannon entropy, a mean pulse peak interval, a root mean square error, and a maximum pulse peak interval;
extracting a feature of the ballistocardiogram signal, where the feature of the ballistocardiogram signal includes at least one of a kurtosis coefficient, a skewness coefficient, a slope, and a height of an H wave of the ballistocardiogram signal; and
determining, based on the feature of the pulse wave signal and the feature of the ballistocardiogram signal, whether the user has atrial fibrillation.

In this embodiment of this application, the feature of the pulse wave signal and the feature of the ballistocardiogram signal are extracted, and then binary classification may be implemented on a heart health status of the user based on a machine learning algorithm. The machine learning algorithm is, for example, a support vector machine SVM, a logistic regression LR, or a k-nearest neighbor KNN algorithm

With reference to the first aspect, in some implementations of the first aspect, the extracting a feature of the ballistocardiogram signal includes:

determining a position of a J wave of the ballistocardiogram signal based on a peak point of the pulse wave signal;
determining a position of the H wave of the ballistocardiogram signal based on the position of the J wave of the ballistocardiogram signal; and
obtaining at least one of the kurtosis coefficient, the skewness coefficient, the slope, and the height of the H wave.

With reference to the first aspect, in some implementations of the first aspect, before the determining, based on the pulse wave signal and the ballistocardiogram signal, whether a heart rhythm of the user is atrial fibrillation, the method further includes:
determining that signal quality of at least one of the acceleration body movement signal, the pulse wave signal, and the ballistocardiogram signal is greater than or equal to a corresponding threshold.

In this way, when atrial fibrillation screening is performed on the user, interference by some factors (such as a movement artifact) to an extracted signal can be avoided, so that accuracy of an atrial fibrillation detection result can be improved.

With reference to the first aspect, in some implementations of the first aspect, the obtaining a ballistocardiogram signal and an acceleration body movement signal based on the acceleration signal includes:

determining a size of a moving window length based on a frequency of a target ballistocardiogram signal and a sampling rate of the target ballistocardiogram signal;

determining the ballistocardiogram signal from the acceleration signal based on the moving window length and the target ballistocardiogram signal; and

determining the acceleration body movement signal based on the acceleration signal and the ballistocardiogram signal.

In this way, in this embodiment of this application, singular spectrum analysis is selected as a zero phase difference filtering algorithm, the BCG signal is extracted from the ACC signal, and after the BCG signal is extracted from the ACC signal, a remaining signal is used as the ACC body movement signal, so that the BCG signal and the ACC body movement signal are extracted.

According to a second aspect, a method for detecting atrial fibrillation is provided. The apparatus is configured to perform the method in any one of the first aspect or the possible implementations of the first aspect. Specifically, the apparatus may include a module configured to perform the method in any one of the first aspect or the possible implementations of the first aspect.

According to a third aspect, a method for detecting atrial fibrillation is provided. The apparatus includes a memory and a processor. The memory is configured to store instructions, and the processor is configured to execute the instructions stored in the memory. In addition, execution of the instructions stored in the memory enables the processor to perform the method in any one of the first aspect or the possible implementations of the first aspect.

According to a fourth aspect, a computer readable storage medium is provided. The computer readable storage medium stores instructions, and when the instructions are run on a computer, the computer performs the method in any one of the first aspect or the possible implementations of the first aspect.

According to a fifth aspect, a computer program product including instructions is provided. When the computer program product is run on a computer, the computer performs the method in any one of the first aspect or the possible implementations of the first aspect.

**BRIEF DESCRIPTION OF DRAWINGS**

FIG. 1 is a schematic diagram of an apparatus for detecting atrial fibrillation according to an embodiment of this application;

FIG. 2 is a schematic flowchart of a method for detecting atrial fibrillation according to an embodiment of this application;

FIG. 3(a) and FIG. 3(b) show examples of a raw PPG signal and a raw ACC signal;

FIG. 4 shows an example of a moving window length L according to an embodiment of this application;

FIG. 5 shows a specific example of a BCG signal;

FIG. 6 shows examples of a target BCG signal and a feature principal component;

FIG. 7 shows examples corresponding to a PPG signal and a BCG signal;

FIG. 8 is a schematic block diagram of an apparatus for detecting atrial fibrillation according to an embodiment of this application; and

FIG. 9 is a schematic block diagram of another apparatus for detecting atrial fibrillation according to an embodiment of this application.

**DESCRIPTION OF EMBODIMENTS**

The following describes technical solutions of this application with reference to accompanying drawings.

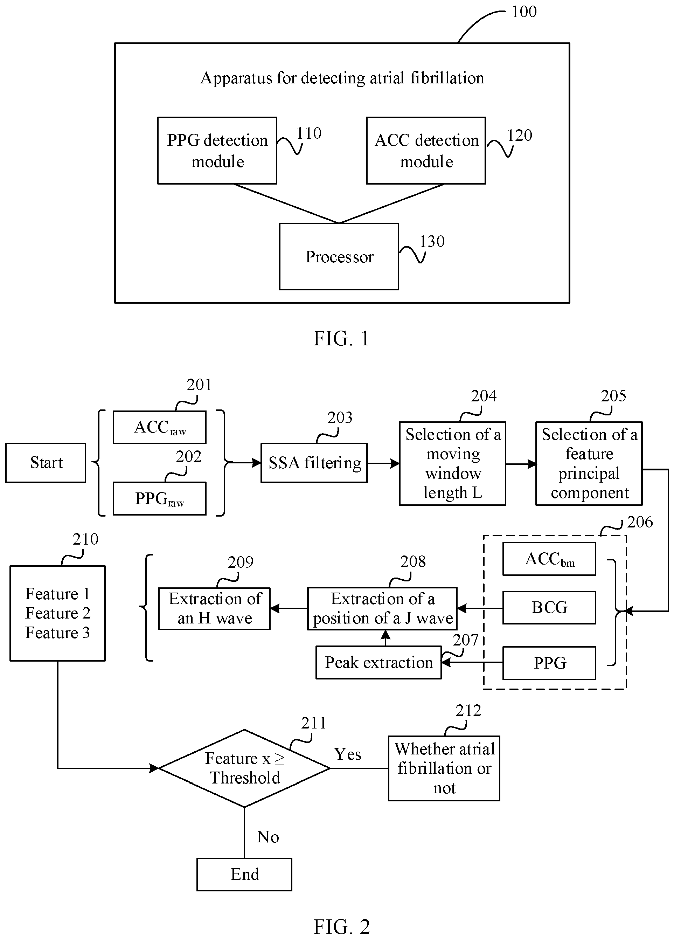

FIG. 1 is a schematic diagram of an apparatus 100 for detecting atrial fibrillation according to an embodiment of this application. In this embodiment of this application, the apparatus for detecting atrial fibrillation may be applied to a terminal device. The terminal device is, for example, a wearable device, such as a sports wristband. This is not limited in this embodiment of this application. As shown in FIG. 1, the apparatus 100 for detecting atrial fibrillation includes a photoplethysmography (Photoplethysmography, PPG) detection module 110, an accelerometer (Accelerometer, ACC) detection module 120, and a processor 130. The PPG detection module may also be referred to as a photoelectric module.

Optionally, the apparatus 100 for detecting atrial fibrillation may further include a communications interface, configured to send, to the processor 130, signals obtained by the PPG detection module 110 and the ACC detection module 120. As an example, the communications interface is, for example, a Bluetooth module, and the processor is, for example, a mobile phone, or a processor in a cloud. This is not limited in this embodiment of this application. Specifically, the PPG detection module 110 is configured to obtain a pulse wave signal of a user. The ACC detection module 120 is configured to obtain an acceleration signal of the user. In this embodiment of this application, the pulse wave signal may also be referred to as a PPG signal, and the acceleration signal may also be referred to as an ACC signal.

The processor 130 is configured to obtain a ballistocardiogram signal and an acceleration body movement signal based on the ACC signal obtained by the ACC detection module 120. In this embodiment of this application, the ballistocardiogram signal may also be referred to as a ballistocardiograph (Ballistocardiograph, BCG) signal, and the acceleration body movement signal may also be referred to as an ACC body movement signal. The BCG signal is used to represent an acceleration change of a body caused by atrial pumping. A waveform shape of the BCG signal is related to an atrial function, for example, related to a P wave in an electrocardiogram. The acceleration body movement signal is used to represent a movement acceleration change of the user, and is a signal with a relatively large amplitude in the ACC signal.

The processor 130 is further configured to determine, based on the pulse wave signal obtained by the PPG detection module 110 and the obtained ballistocardiogram signal, whether the user has atrial fibrillation.

Therefore, in this embodiment of this application, the PPG signal and the ACC signal are obtained, the BCG signal and the ACC body movement signal are obtained based on the ACC signal, and then whether the user has atrial fibrillation is screened based on the BCG signal and the PPG signal. Because the BCG signal can represent the acceleration change of the body caused by atrial pumping, the BCG signal is introduced during atrial fibrillation screening in this embodiment of this application, to help improve accuracy of atrial fibrillation prediction.

FIG. 2 is a schematic flowchart of a method for detecting atrial fibrillation according to an embodiment of this application. It should be understood that FIG. 2 shows steps or operations of a service processing method, but these steps or operations are merely examples. In this embodiment of this application, another operation or a variation of the operations in FIG. 2 may be further performed. In addition, the steps in FIG. 2 may be performed in an order different from that presented in FIG. 2, and it is possible that not all of the operations in FIG. 2 are to be performed. 201. Obtain a raw ACC signal (which may be represented as $ACC_{raw}$). Specifically, a raw ACC signal of a user may be detected by using an ACC detection module 120. In an example, a raw ACC signal with duration of one minute (min) may be obtained.

202. Obtain a raw PPG signal (which may be represented as $PPG_{raw}$). Specifically, a raw PPG signal of the user may be detected by using a PPG detection module 110. In an example, a raw ACC signal with duration of one minute (min) may be obtained.

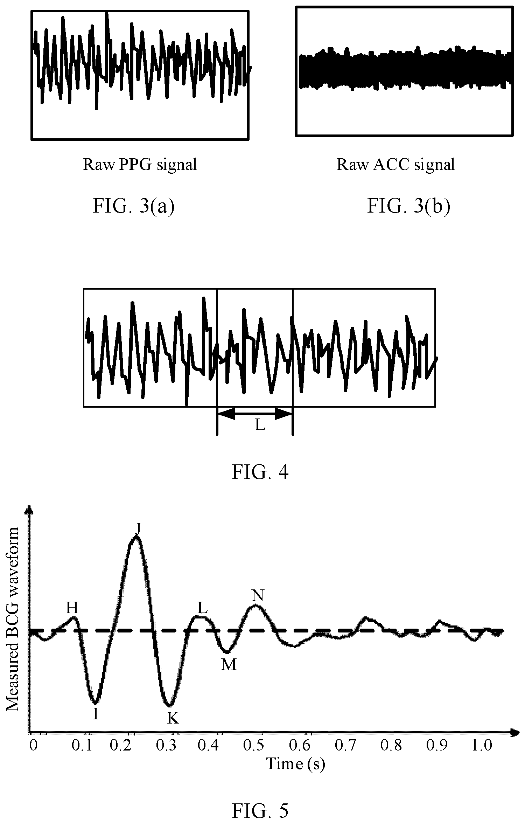

An example of the raw PPG signal is illustrated in FIG. 3(a) on the left, and an example of the raw ACC signal is illustrated in FIG. 3(b) on the right.

In an embodiment of this application, a BCG signal and an ACC body movement signal may be obtained based on the ACC signal. As an example, 203 to 205 show an example in which the BCG signal and the ACC body movement signal are obtained based on the ACC signal.

203. For the ACC signal obtained in 202, select singular spectrum analysis (singular spectrum analysis, SSA) as a zero phase difference filtering algorithm, that is, SSA filtering.

204. Select a moving window length L.

Specifically, a size of the moving window length L may be determined based on a frequency (for example, $(f_1, f_2, f_3)$) of a target BCG signal and a sampling rate ($f_s$) of the target BCG signal, that is, $L=F(f_s, f_1, f_2, f_3)$. FIG. 4 shows an example of a moving window length L according to an embodiment of this application. Herein, the target BCG signal is a template used during SSA filtering, and may be a sample of any BCG signal obtained in advance. FIG. 5 shows a specific example of a BCG signal. As shown in FIG. 5, the BCG signal includes a J wave, an H wave, an I wave, a K wave, an L wave, an M wave, and an N wave. The J wave is a maximum peak point in the BCG signal, and the H wave is a peak point before the J wave. The H wave is related to a P wave in an electrocardiogram.

205. Select a feature principal component.

In an optional embodiment, the ballistocardiogram signal may be extracted from the acceleration signal based on the moving window length L and the target BCG signal.

Specifically, the ACC signal may be divided into several signals, that is, several feature principal components, based on the moving window length L. Because the moving window length L is related to the frequency and the sampling rate of the target BCG signal, spectral densities between data of the several feature principal components are correlated. As an example, the several feature principal components may belong to different frequency bands.

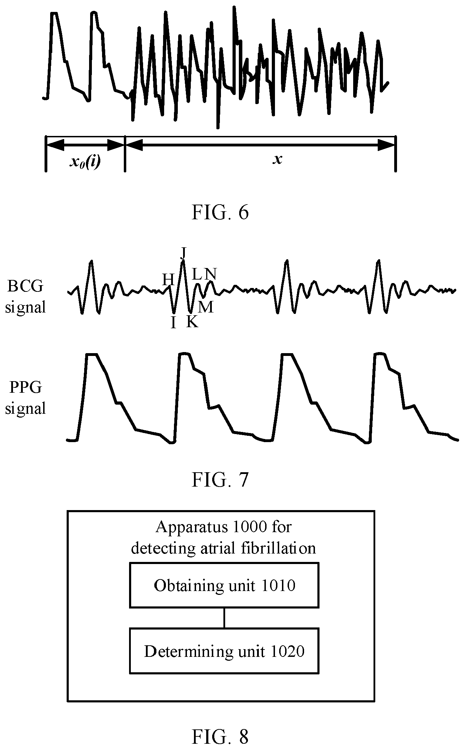

Then, as shown in FIG. 6, a signal $x + x_0(i)$ may be obtained by adding a sample component (that is, a signal of an $x_0(i)$ segment in FIG. 6) of the target BCG signal before a feature principal component (that is, a signal of an $x$ segment in FIG. 6). In this way, a proportion of the target BCG signal in the raw ACC signal increases, and a corresponding feature principal component with high correlation with the target BCG signal is placed in the front. Therefore, based on the foregoing principle, a function G may be designed, and a feature principal component PC may be selected based on the following formula:

$$PC=G(x + x_0(i)).$$

206. Obtain a BCG signal, an ACC body movement signal (which may be represented as $ACC_{bm}$), and a PPG signal. Specifically, the feature principal component PC selected based on the target BCG signal in 205 is a BCG signal extracted based on the ACC signal.

Then, the $ACC_{bm}$ signal is obtained based on the extracted BCG signal and the ACC signal. As an example, after the BCG signal is extracted from the ACC signal, a remaining signal may be the ACC body movement signal.

In an example, in this embodiment of this application, obtaining a PPG signal based on the $PPG_{raw}$ signal may be specifically performing noise reduction processing on the $PPG_{raw}$ signal by using a filter, and a signal obtained after the processing is the PPG signal.

Optionally, in this embodiment of this application, noise reduction processing may be performed on the $ACC_{raw}$ signal. Specifically, after the $ACC_{raw}$ signal is obtained, noise reduction processing may be performed on the $ACC_{raw}$ signal, or noise reduction processing may be performed on the ACC signal after the BCG signal is extracted. This is not limited in this embodiment of this application.

207. Perform peak extraction on the PPG signal.

208. Determine a position of a J wave of the BCG signal based on a peak point of the PPG signal.

As shown in FIG. 7, the peak point of the PPG signal corresponds to the position of the J wave of the BCG signal. 209. Determine a position of an H wave of the BCG signal based on the position of the J wave of the BCG signal. Specifically, the first peak on the left side of the J wave of the BCG signal may be determined as the H wave.

210. Separately extract features of the $ACC_{bm}$ signal, the BCG signal, and the PPG signal.

As an example, at least one of kurtosis, a standard deviation, and Shannon entropy of the $ACC_{bm}$ signal may be extracted as a feature of the $ACC_{bm}$ signal. The feature of the $ACC_{bm}$ signal may be denoted as a feature 1 (feat 1). As an example, at least one of a kurtosis coefficient, a skewness coefficient, a slope, and a height of the H wave of the BCG signal may be extracted as a feature of the BCG signal. The feature of the BCG signal may be denoted as a feature 2 (feat 2).

As an example, at least one of Shannon entropy, a mean pulse peak interval (mean RR), a root mean square error (RMSSD), and a maximum pulse peak interval of the PPG signal may be extracted as a feature of the PPG signal. The feature of the PPG signal may be denoted as a feature 3 (feat 3).

In this embodiment of this application, whether the user has atrial fibrillation may be determined based on the feature of the BCG signal and the feature of the PPG signal.

Optionally, before whether the user has atrial fibrillation is determined based on the feature of the BCG signal and the feature of the PPG signal, whether a feature x is greater than or equal to a threshold may be further determined based on whether signal quality of at least one of the $ACC_{bm}$ signal, the BCG signal, and the PPG signal is greater than or equal to a specific threshold, that is, 211 in FIG. 2, where x=1, 2, and 3.

That is, whether signal quality of a signal obtained in this test meets a calculation requirement is determined. Specifically, in this embodiment of this application, detection of the PPG signal and the BCG signal may be interfered by a movement artifact. Whether interference by a movement artifact to a signal extracted in a test affects accuracy of a detection result may be determined by comparing at least one of the $ACC_{bm}$ signal, the BCG signal, and the PPG signal with a corresponding threshold value. That is, when the movement artifact has no obvious influence on the detection result, whether atrial fibrillation or not may be determined based on the BCG signal and the PPG signal. Accuracy of atrial fibrillation screening decreases when the interference by the movement artifact to the detection result is relatively great.

When the feature x is greater than or equal to the corresponding threshold, 212 is to be performed. When the feature x is less than or equal to the corresponding threshold, the detection is to be stopped.

As an example, the feature x may be the feature 1, that is, the feature of $ACC_{bm}$. That is, when quality of the $ACC_{bm}$ signal determined based on the feature of $ACC_{bm}$ is greater than a threshold corresponding to the $ACC_{bm}$ signal, 212 is to be performed; or otherwise, the detection is to be stopped.

As another example, the feature x may be the feature 1 and the feature 2, that is, the feature of $ACC_{bm}$ and the feature of the BCG signal. That is, when quality of the $ACC_{bm}$ signal determined based on the feature of $ACC_{bm}$ is greater than a threshold corresponding to the $ACC_{bm}$ signal, and quality of the BCG signal determined based on the feature of the BCG signal is greater than a threshold corresponding to the BCG signal, 212 is to be performed; or otherwise, the detection is to be stopped.

As another example, the feature x may be the feature 1, the feature 2, and the feature 3, that is, the feature of $ACC_{bm}$, the feature of the BCG signal, and the feature of the PPG signal. That is, when quality of the $ACC_{bm}$ signal determined based on the feature of $ACC_{bm}$ is greater than a threshold corresponding to the $ACC_{bm}$ signal, quality of the BCG signal determined based on the feature of the BCG signal is greater than a threshold corresponding to the BCG signal, and quality of the PPG signal determined based on the feature of the PPG signal is greater than a threshold corresponding to the PPG signal, 212 is to be performed; or otherwise, the detection is to be stopped.

212. Determine whether atrial fibrillation or not based on the PPG signal and the BCG signal.

As an example, based on the feature of the PPG signal and the feature of the BCG signal, binary classification may be implemented on a heart health status of the user based on a machine learning algorithm, that is, whether a heart rhythm of the user is normal or atrial fibrillation may be determined. Herein, the machine learning algorithm is, for example, a support vector machine (support vector machine, SVM), a logistic regression (logistic regression, LR), or a k-nearest neighbor (k-nearest neighbor, KNN) algorithm. This is not limited in this embodiment of this application. Therefore, in this embodiment of this application, the PPG signal and the ACC signal are obtained, the BCG signal and the ACC body movement signal are obtained based on the ACC signal, and then whether the user has atrial fibrillation is screened based on the BCG signal and the PPG signal. Because the BCG signal can represent an acceleration change of a body caused by atrial pumping, the BCG signal is introduced during atrial fibrillation screening in this embodiment of this application, to help improve accuracy of atrial fibrillation prediction.

FIG. 8 is a schematic block diagram of an apparatus 1000 for detecting atrial fibrillation according to an embodiment of this application. The apparatus for atrial fibrillation screening includes an obtaining unit 1010 and a determining unit 1020.

The obtaining unit 1010 is configured to obtain a pulse wave signal and an acceleration signal of a user.

The determining unit 1020 is configured to determine a ballistocardiogram signal and an acceleration body movement signal based on the acceleration signal, where the ballistocardiogram signal is used to represent an acceleration change of a body caused by atrial pumping, and the acceleration body movement signal is used to represent a movement acceleration change of the user.

The determining unit 1020 is further configured to determine, based on the pulse wave signal and the ballistocardiogram signal, whether the user has atrial fibrillation.

Therefore, in this embodiment of this application, a PPG signal and an ACC signal are obtained, a BCG signal and an ACC body movement signal are obtained based on the ACC signal, and then whether the user has atrial fibrillation is screened based on the BCG signal and the PPG signal. Because the BCG signal can represent the acceleration change of the body caused by atrial pumping, the BCG signal is introduced during atrial fibrillation screening in this embodiment of this application, to help improve accuracy of atrial fibrillation prediction.

Optionally, the determining unit 1020 is specifically configured to:

extract a feature of the pulse wave signal, where the feature of the pulse wave signal includes at least one of Shannon entropy, a mean pulse peak interval, a root mean square error, and a maximum pulse peak interval;
extract a feature of the ballistocardiogram signal, where the feature of the ballistocardiogram signal includes at least one of a kurtosis coefficient, a skewness coefficient, a slope, and a height of an H wave of the ballistocardiogram signal; and
determine, based on the feature of the pulse wave signal and the feature of the ballistocardiogram signal, whether the user has atrial fibrillation.

Optionally, the determining unit 1020 is specifically configured to:

determine a position of a J wave of the ballistocardiogram signal based on a peak point of the pulse wave signal;
determine a position of the H wave of the ballistocardiogram signal based on the position of the J wave of the ballistocardiogram signal; and
obtain at least one of the kurtosis coefficient, the skewness coefficient, the slope, and the height of the H wave.

Optionally, the determining unit 1020 is further configured to:
determine that signal quality of at least one of the acceleration body movement signal, the pulse wave signal, and the ballistocardiogram signal is greater than or equal to a corresponding threshold.

Optionally, the determining unit 1020 is specifically configured to:

determine a size of a moving window length based on a frequency of a target ballistocardiogram signal and a sampling rate of the target ballistocardiogram signal;
determine the ballistocardiogram signal from the acceleration signal based on the moving window length and the target ballistocardiogram signal; and
determine the acceleration body movement signal based on the acceleration signal and the ballistocardiogram signal.

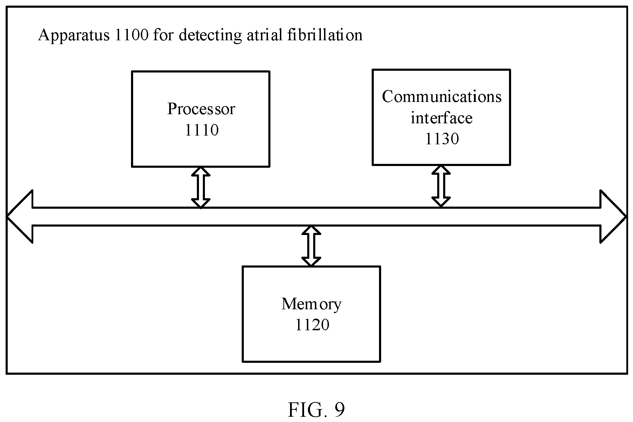

It should be noted that, in this embodiment of this application, the obtaining unit 1010 may be implemented by a communications interface, and the determining unit 1020 may be implemented by a processor. As shown in FIG. 9, an apparatus 1100 for detecting atrial fibrillation may include a processor 1110, a memory 1120, and a communications interface 1130. The memory 1120 may be configured to store code or the like to be executed by the processor 1110, and the processor 1110 may be configured to process data or a program.

In an implementation process, steps in the foregoing methods can be implemented by using a hardware integrated logical circuit in the processor 1110, or by using instructions in a form of software. The steps of the method disclosed with reference to the embodiments of the present invention may be directly performed by a hardware processor, or may be performed by using a combination of hardware in the processor and a software module. A software module may be located in a mature storage medium in the art, such as a random access memory, a flash memory, a read-only memory, a programmable read-only memory, an electrically erasable programmable memory, or a register. The storage medium is located in the memory 1120, and the processor 1110 reads information in the memory 1120 and completes the steps in the foregoing methods in combination with hardware of the processor. To avoid repetition, details are not described herein again.

The apparatus 1000 shown in FIG. 8 or the apparatus 1100 shown in FIG. 9 can implement processes of the method for detecting atrial fibrillation corresponding to the foregoing method embodiment. Specifically, for the apparatus 1000 or the apparatus 1100, refer to the foregoing description. To avoid repetition, details are not described herein again.

An embodiment of this application further provides a computer readable medium, configured to store a computer program. The computer program includes instructions used to perform a corresponding method in the foregoing method embodiment.

An embodiment of this application further provides a computer program product. The computer program product includes computer program code. When a processor of an apparatus tested by the computer program code runs, the apparatus for avoiding packet fragmentation performs a corresponding method in the foregoing method embodiment. The embodiments in this application may be used independently, or may be used jointly. This is not limited herein.

It should be understood that descriptions such as first and second in the embodiments of this application are merely used to represent and distinguish between described objects, do not represent a sequence or indicate a special limitation on a quantity of devices in the embodiments of this application, and cannot constitute any limitation on the embodiments of this application.

It should be further understood that sequence numbers of the foregoing processes do not mean execution sequences in various embodiments of this application. The execution sequences of the processes should be determined according to functions and internal logic of the processes, and should not be construed as any limitation on the implementation processes of the embodiments of this application.

A person of ordinary skill in the art may be aware that, in combination with the examples described in the embodiments disclosed in this specification, units and algorithm steps may be implemented by electronic hardware or a combination of computer software and electronic hardware. Whether the functions are performed by hardware or software depends on particular applications and design constraint conditions of the technical solutions. A person skilled in the art may use different methods to implement the described functions for each particular application, but it should not be considered that the implementation goes beyond the scope of this application.

It may be clearly understood by a person skilled in the art that, for the purpose of convenient and brief description, for a detailed working process of the foregoing system, apparatus, and unit, refer to a corresponding process in the foregoing method embodiments, and details are not described herein again.

In the several embodiments provided in this application, it should be understood that the disclosed system, apparatus, and method may be implemented in other manners. For example, the described apparatus embodiment is merely an example. For example, the division into the units is merely logical function division and may be other division in actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electronic, mechanical, or other forms.

The units described as separate parts may or may not be physically separate, and parts displayed as units may or may not be physical units, may be located in one position, or may be distributed on a plurality of network units. Some or all of the units may be selected based on actual requirements to achieve the objectives of the solutions of the embodiments.

In addition, functional units in the embodiments of this application may be integrated into one processing unit, or each of the units may exist alone physically, or two or more units are integrated into one unit.

When the functions are implemented in the form of a software functional unit and sold or used as an independent product, the functions may be stored in a computer-readable storage medium. Based on such an understanding, the technical solutions of this application essentially, or the part contributing to the current technology, or some of the technical solutions may be implemented in a form of a software product. The software product is stored in a storage medium, and includes several instructions for instructing a computer device (which may be a personal computer, a server, or a network device) to perform all or some of the steps of the methods described in the embodiments of this application. The foregoing storage medium includes: any medium that can store program code, such as a USB flash drive, a removable hard disk, a read-only memory (read-only memory, ROM), a random access memory (random access memory, RAM), a magnetic disk, or an optical disc.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

**Claims**

1.   A method for detecting atrial fibrillation, comprising:

      obtaining a pulse wave signal and an acceleration signal of a user;
      determining a ballistocardiogram signal and an acceleration body movement signal based on the acceleration signal, wherein the ballistocardiogram signal is used to represent an acceleration change of a body caused by atrial pumping, and the acceleration body movement signal is used to represent a movement acceleration change of the user; and
determining, based on the pulse wave signal and the ballistocardiogram signal, whether the user has atrial fibrillation.

2. The method according to claim 1, wherein the determining, based on the pulse wave signal and the ballistocardiogram signal, whether the user has atrial fibrillation comprises:

extracting a feature of the pulse wave signal, wherein the feature of the pulse wave signal comprises at least one of Shannon entropy, a mean pulse peak interval, a root mean square error, and a maximum pulse peak interval;
extracting a feature of the ballistocardiogram signal, wherein the feature of the ballistocardiogram signal comprises at least one of a kurtosis coefficient, a skewness coefficient, a slope, and a height of an H wave of the ballistocardiogram signal; and
determining, based on the feature of the pulse wave signal and the feature of the ballistocardiogram signal, whether the user has atrial fibrillation.

3. The method according to claim 2, wherein the extracting a feature of the ballistocardiogram signal comprises:

determining a position of a J wave of the ballistocardiogram signal based on a peak point of the pulse wave signal;
determining a position of the H wave of the ballistocardiogram signal based on the position of the J wave of the ballistocardiogram signal; and
obtaining at least one of the kurtosis coefficient, the skewness coefficient, the slope, and the height of the H wave.

4. The method according to any one of claims 1 to 3, wherein before the determining, based on the pulse wave signal and the ballistocardiogram signal, whether a heart rhythm of the user is atrial fibrillation, the method further comprises:
determining that signal quality of at least one of the acceleration body movement signal, the pulse wave signal, and the ballistocardiogram signal is greater than or equal to a corresponding threshold.

5. The method according to any one of claims 1 to 4, wherein the obtaining a ballistocardiogram signal and an acceleration body movement signal based on the acceleration signal comprises:

determining a size of a moving window length based on a frequency of a target ballistocardiogram signal and a sampling rate of the target ballistocardiogram signal;
determining the ballistocardiogram signal from the acceleration signal based on the moving window length and the target ballistocardiogram signal; and
determining the acceleration body movement signal based on the acceleration signal and the ballistocardiogram signal.

6. An apparatus for detecting atrial fibrillation, comprising:

an obtaining unit, configured to obtain a pulse wave signal and an acceleration signal of a user; and
a determining unit, configured to determine a ballistocardiogram signal and an acceleration body movement signal based on the acceleration signal, wherein the ballistocardiogram signal is used to represent an acceleration change of a body caused by atrial pumping, and the acceleration body movement signal is used to represent a movement acceleration change of the user; and
the determining unit is further configured to determine, based on the pulse wave signal and the ballistocardiogram signal, whether the user has atrial fibrillation.

7. The apparatus according to claim 6, wherein the determining unit is specifically configured to:

extract a feature of the pulse wave signal, wherein the feature of the pulse wave signal comprises at least one of Shannon entropy, a mean pulse peak interval, a root mean square error, and a maximum pulse peak interval;
extract a feature of the ballistocardiogram signal, wherein the feature of the ballistocardiogram signal comprises at least one of a kurtosis coefficient, a skewness coefficient, a slope, and a height of an H wave of the ballistocardiogram signal; and
determine, based on the feature of the pulse wave signal and the feature of the ballistocardiogram signal, whether the user has atrial fibrillation.

8. The apparatus according to claim 7, wherein the determining unit is specifically configured to:

determine a position of a J wave of the ballistocardiogram signal based on a peak point of the pulse wave signal;

determine a position of the H wave of the ballistocardiogram signal based on the position of the J wave of the ballistocardiogram signal; and

obtain at least one of the kurtosis coefficient, the skewness coefficient, the slope, and the height of the H wave.

9. The apparatus according to any one of claims 6 to 8, wherein the determining unit is further configured to:

determine that signal quality of at least one of the acceleration body movement signal, the pulse wave signal, and the ballistocardiogram signal is greater than or equal to a corresponding threshold.

10. The apparatus according to any one of claims 6 to 9, wherein the determining unit is specifically configured to:

determine a size of a moving window length based on a frequency of a target ballistocardiogram signal and a sampling rate of the target ballistocardiogram signal;

determine the ballistocardiogram signal from the acceleration signal based on the moving window length and the target ballistocardiogram signal; and

determine the acceleration body movement signal based on the acceleration signal and the ballistocardiogram signal.

100

Apparatus for detecting atrial fibrillation

| PPG detection module | 110 | | ACC detection module | 120 |

Processor 130

FIG. 1

Start

{ ACC$_{raw}$ 201

PPG$_{raw}$ 202 }

SSA filtering 203

Selection of a moving window length L 204

Selection of a feature principal component 205

206

ACC$_{bm}$

BCG

PPG

207

Peak extraction

Extraction of a position of a J wave 208

Extraction of an H wave 209

Feature 1
Feature 2
Feature 3
210

Feature x ≥ Threshold 211

Yes → Whether atrial fibrillation or not 212

No

End

FIG. 2

Raw PPG signal

FIG. 3(a)

Raw ACC signal

FIG. 3(b)

FIG. 4

FIG. 5

FIG. 6

FIG. 7

FIG. 8

Apparatus 1100 for detecting atrial fibrillation

Processor
1110

Communications
interface
1130

Memory
1120

FIG. 9

## INTERNATIONAL SEARCH REPORT

| International application No. |
| --- |
| **PCT/CN2019/074670** |

| A. | CLASSIFICATION OF SUBJECT MATTER |
| --- | --- |

A61B 5/00(2006.01)i

According to International Patent Classification (IPC) or to both national classification and IPC

| B. | FIELDS SEARCHED |
| --- | --- |

Minimum documentation searched (classification system followed by classification symbols)

A61B 5/-

Documentation searched other than minimum documentation to the extent that such documents are included in the fields searched

Electronic data base consulted during the international search (name of data base and, where practicable, search terms used)

CNKI, CNPAT, WPI, EPODOC: 华为, 杨斌, 李宏宝, 张杰, 脉搏波, 脉率, 脉波, 加速度, 加速计, 心冲击, 心动描记, 房颤, 心房纤颤, 心律, 失常, 紊乱, 异常, AF, pulse wave, accelerated speed, accelerated velocity, acceleration, BCG, ballistocardiogra+, auricular fibrillation, arrhythmia

| C. | DOCUMENTS CONSIDERED TO BE RELEVANT | |
| --- | --- | --- |
| Category* | Citation of document, with indication, where appropriate, of the relevant passages | Relevant to claim No. |
| Y | CN 104837401 A (KONINKLIJKE PHILIPS N.V.) 12 August 2015 (2015-08-12) description, paragraphs [0016], [0017], [0019]-[0021], [0024]-[0029] and [0038], and claim 1 | 1-10 |
| Y | CN 108606798 A (NORTHEASTERN UNIVERSITY) 02 October 2018 (2018-10-02) description, paragraph [0042] | 1-10 |
| A | CN 105902265 A (GUANGDONG LIFESENSE MEDICAL ELECTRONIC CO., LTD.) 31 August 2016 (2016-08-31) entire document | 1-10 |
| A | CN 104352229 A (ICARETECH HEALTHCARE CO., LTD.) 18 February 2015 (2015-02-18) entire document | 1-10 |
| A | CN 109199327 A (SAMSUNG ELECTRONICS CO., LTD.) 15 January 2019 (2019-01-15) entire document | 1-10 |
| A | US 10105547 B2 (WEST AFFUM HOLDINGS CORP.) 23 October 2018 (2018-10-23) entire document | 1-10 |

☐ Further documents are listed in the continuation of Box C.  ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **12 October 2019** | **04 November 2019** |

| Name and mailing address of the ISA/CN | Authorized officer |
| --- | --- |
| **China National Intellectual Property Administration (ISA/CN)** **No. 6, Xitucheng Road, Jimenqiao Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2019/074670**

| Patent document cited in search report | | | Publication date (day/month/year) | Patent family member(s) | | | Publication date (day/month/year) |
|---|---|---|---|---|---|---|---|
| CN | 104837401 | A | 12 August 2015 | JP | 2016504085 | A | 12 February 2016 |
| | | | | RU | 2655443 | C2 | 28 May 2018 |
| | | | | JP | 6335920 | B2 | 30 May 2018 |
| | | | | WO | 2014091382 | A1 | 19 June 2014 |
| | | | | CA | 2894396 | A1 | 19 June 2014 |
| | | | | US | 2015305684 | A1 | 29 October 2015 |
| | | | | RU | 2015128271 | A | 23 January 2017 |
| | | | | MX | 2015007282 | A | 12 August 2015 |
| | | | | CN | 104837401 | B | 12 September 2017 |
| | | | | EP | 2931120 | A1 | 21 October 2015 |
| CN | 108606798 | A | 02 October 2018 | | None | | |
| CN | 105902265 | A | 31 August 2016 | CN | 105902265 | B | 19 July 2019 |
| CN | 104352229 | A | 18 February 2015 | CN | 104352229 | B | 26 April 2017 |
| CN | 109199327 | A | 15 January 2019 | KR | 20190003325 | A | 09 January 2019 |
| | | | | US | 2019000332 | A1 | 03 January 2019 |
| US | 10105547 | B2 | 23 October 2018 | US | 2019091481 | A1 | 28 March 2019 |
| | | | | US | 2017128735 | A1 | 11 May 2017 |

Form PCT/ISA/210 (patent family annex) (January 2015)